# EUROPEAN PATENT SPECIFICATION

(11) **EP 1 544 213 B1**
(45) Date of publication and mention of the grant of the patent: **20.02.2008**
(21) Application number: 03029464.9
(22) Date of filing: 19.12.2003
(51) Int. Cl.: C07K 16/28, A61K 39/395, G01N 33/53

(54) **Use of ligands of the antigen cd52 for the treatment of solid tumours and bone-related cancerous diseases**
Verwendung von Liganden von CD52 Antigen zur Behandlung von soliden Tumoren und von Knochenkrebserkrankungen
Utilisation de ligands de l'antigène CD52 pour le traitement de tumeurs solides et des cancers de type osseux

(43) Date of publication of application: 22.06.2005
(73) Proprietor: Charité - Universitätsmedizin Berlin, 10117 Berlin (DE)
(72) Inventor: Krenn, Veit, Prof. Dr., 13503 Berlin (DE)
(74) Representative: Krauss, Jan

(56) References cited:
- WO-A-03/050257
- DE KROON ET AL.: "Anti-CD45 and anti-CD52 (Campath) monoclonal antibodies effectively eliminate systemically disseminated human non-Hodgkin's lymphoma B cells in Scid mice" EXPERIMENTAL HEMATOLOGY, vol. 24, 1996, pages 919-926, XP008030315
- TONE ET AL: "Structure and chromosomal location of mouse and human CD52 genes" BIOCHEMICA AND BIOPHYSICA ACTA, vol. 1446, 1999, pages 334-340, XP002278471
- A. DOMAGALA ET AL.: "CD52 antigen - a review" MED SCI MONIT, vol. 7, no. 2, 2001, pages 325-331, XP001190887
- WORTH LL ET AL.: "Biologic response modifiers in pediatric cancer" PEDIATRIC ONCOLOGY, vol. 15, no. 4, August 2001 (2001-08), pages 723-740, XP008030314

## Description

The present invention relates to the use of a CD52-specific antibody for the preparation of a medicament for the treatment of CD52-expressing solid bone tumours.

### Description

Human CD52 is a glycosylphosphatidylinositol (GPI)-anchored antigen expressed on the all lymphocytes as well as within the male genital tract, where it can be produced by epithelial cells of the distal epididymis and duct deferens. The physiological role of CD52 on lymphocytes is unclear, although antibody directed to CD52, CAMPATH-1, is capable of complement activation and it can be clinically useful to treat lympho-proliferative disorders and to deplete lymphocytes in bone marrow transplants (Domagala A, Kurpisz M. CD52 antigen - a review. Med Sci Monit. 2001 Mar-Apr;7(2):325-31).

Presently, CD52-specific antibodies are used in B-cell chronic lymphocytic leukaemia (B-CLL) (Moreton P, Hillmen P. Alemtuzumab therapy in B-cell lymphoproliferative disorders. Semin Oncol. 2003 Aug;30(4):493-501; Lundin J, et al. Phase II trial of subcutaneous anti-CD52 monoclonal antibody alemtuzumab (Campath-1H) as first-line treatment for patients with B-cell chronic lymphocytic leukaemia (B-CLL). Blood. 2002 Aug 1;100(3):768-73.). The efficiency of the therapy correlates with the quantity of the CD52 expression (D'Arena G, et al. Quantitative evaluation of CD52 expression in B-cell chronic lymphocytic leukaemia. Leuk Lymphoma. 2003 Jul;44(7): 1255-7.). The CD52 antibody is furthermore used in the therapy of transplantation rejection (Hale G, et al. CD52 antibodies for prevention of graft-versus-host disease and graft rejection following transplantation of allogeneic peripheral blood stem cells. Bone Marrow Transplant. 2000 Jul;26(1):69-76.) and, most recently, in Behcet's disease (Lockwood CM, Hale G, Waldman H, Jayne DR. Remission induction in Behcet's disease following lymphocyte depletion by the anti-CD52 antibody CAMPATH 1-H. Rheumatology (Oxford). 2003 Aug 29).

Alemtuzumab (Campath-1H, Ilex Pharmaceuticals, San Antonio, TX) is a humanised monoclonal antibody that recognises the CD52 antigen expressed on malignant and normal B lymphocytes. It has come to be used therapeutically in B-cell malignancies. Responses are seen in non-Hodgkin's lymphoma (NHL), and alemtuzumab can induce molecular remissions in relapsed chronic lymphocytic leukaemia (CLL), even when refractory to purine analogues. Most studies reveal the responses to be superior in the absence of bulky disease. Infusion-related side effects such as rigors, hypotension, and nausea are reduced by using the subcutaneous route of administration. Infectious complications are the most important toxicity seen and are related to the depletion of normal lymphocytes. The clinical efficacy in combination with both fludarabine and rituximab is under investigation.

Alemtuzumab is an unconjugated, humanised, monoclonal antibody directed against the cell surface antigen CD52 on lymphocytes and monocytes. In noncomparative phase I/II studies in patients with B-cell chronic lymphocytic leukaemia (B-CLL) relapsed after or refractory to alkylating agents and fludarabine, intravenous (IV) administration of alemtuzumab 30 mg/day three times weekly for up to 12 weeks was associated with overall objective response (OR) rates of 21-59% (Frampton JE, Wagstaff AJ. Alemtuzumab. Drugs. 2003;63(12):1229-43; discussion 1245-6).

The biological effect of ligands of CD52 seems to be mediated by an unusual lytic effect on the target cells (Kirk AD, et al. Results from a human renal allograft tolerance trial evaluating the humanized CD52-specific monoclonal antibody alemtuzumab (CAMPATH-1H). Transplantation. 2003 Jul 15;76(1): 120-9).

CA 2,060,384 describes a method of treatment of a human or animal subject suffering from T-cell mediated inflammation of the joints, such as rheumatoid arthritis, which comprises administering to said subject an effective amount of an antibody recognising the CDw52 antigen. Preferably the antibody is CAMPATH-1H administered in an amount of 1 to about 100mg per day for a period between 1 and 30 days.

WO 93/01296 describes a recombinant vaccinia virus capable of expressing on infection of a suitable cell the light chain and/or the heavy chain of an antibody and the use of such a virus in the production of recombinant antibodies. The antibody may be a chimaeric or a humanised (CDR-grafted) antibody for example a humanised antibody against the CDw52 antigen.

WO 93/08837 describes a stabilised immunoglobulin composition comprising at least one immunoglobulin together with a stabilising amount of a chelator of copper ions such as EDTA or citrate. Preferably the immunoglobulin is an antibody, for example a recombinant CDR-grafted antibody against the CDw52 antigen, most preferably CAMPATH-1H.

CH 681305 describes a purified preparation of an anti-CDW52 antibody which exhibits on size exclusion chromatography: a single peak under non-reducing conditions and two major peaks under reducing conditions. Also a process of purifying an anti-CDW52 antibody, formulations containing such a purified preparation and uses thereof are described.

US 5,786,176 and WO 94/02604 describe recombinant cell lines, in particular mammalian cell lines, capable of expressing recombinant CDw52 antigen or an antigenic fragment thereof. The cell lines can be used for the production of recombinant CDw52 antigen and in assaying for anti-CDw52 antibody in a sample.

US 6,120,766 and WO 93/10817 describe the use of an antibody recognising the CDw52 antigen in the treatment of multiple sclerosis. Preferably, the antibody is the humanised antibody in the humanised antibody CAMPATH-1H.

WO 93/07899 describes the use of an antibody recognising the CDw52 antigen for the treatment of T-cell mediated inflammation of the joints, in particular for the treatment of rheumatoid arthritis. Preferably the antibody is the humanised antibody CAMPATH-1H which may be used together with another immunosuppressive antibody or an immunosuppressive agent such as a steroid.

WO 03/050257 describes a method for targeting a molecule to a CD52-expressing leukaemia cell by a) increasing the expression of the CD52 by contacting the cell with all-trans-retinoic cell, and b) contacting the cell with anti-CD52-antibody. WO 03/050257 does not describe solid tumour cells and their use in CD52-ligand-screening.

De Kroon et al. (de Kroon JF, de Paus RA, Kluin-Nelemans HC, Kluin PM, van Bergen CA, Munro AJ, Hale G, Willemze R, Falkenburg JH. Anti-CD45 and anti-CD52 (Campath) monoclonal antibodies effectively eliminate systematically disseminated human non-Hodgkin's lymphoma B cells in Scid mice. Exp Hematol. 1996 Jul;24(8):919-26.) describe experiments in mice, into which human DoHH2 or BEVA-lymphoma cells have been injected. The surviving mice, when injected with high numbers of BEVA cells, after treatment with anti-CD52-antibody still exhibit tumor masses in liver, kidney and mesenteric lymph nodes. Thus, the results are discussed as indicating that treatment with anti-CD52 mAbs potentially may be useful only as adjuvant immunotherapy for systemically disseminated B cell lymphoma.

Solid tumours make up about 30% of all paediatric cancers. The most common types of solid tumours in children include brain tumours, neuroblastoma, rhabdomyosarcoma, Wilms' tumor, and osteosarcoma (see, for example, Kline NE, Sevier N. Solid tumors in children. J Pediatr Nurs. 2003 Apr;18(2):96-102.). As one additional example, high grade periacetabular osteosarcomas extending to the sacro-iliac region present a difficult management problem. Functional outcome is poor and the prognosis for this group of patients is not improved by surgery (Pollock RC, et al. The swing procedure for pelvic ring reconstruction following tumour excision. Eur J Surg Oncol. 2003 Feb;29(1):59-63).

Document WO 03/080 671 discloses anti-RANK antibodies for the treatment of primary bone tumour.

Giant cell tumours represent approximately 5% of all primary bone tumours, which typically can be found in hollow bones (joint hub) and in flat bones (pelvic belt). Therapeutically these tumours can pose particular problems in that they can be localised in areas in which they can not be removed (e.g. sacral bone, cranial skeleton). Treatment of giant-cell tumour of bone generally involves wide en bloc resection of the lesion and the surrounding bone or curettage with or without bone-grafting or the use of cement. Radiation therapy has been used for patients who cannot be operated on for medical reasons or who have a tumour that is technically difficult to resect or that cannot be resected because of its location (Chakravarti A, et al. Megavoltage radiation therapy for axial and inoperable giant-cell tumor of bone. J Bone Joint Surg Am. 1999 Nov;81(11):1566-73).

For all of the above discussed bone-related cancers, alternative treatment options are needed. It is therefore an object of the present invention, to provide new treatment options for those bone-related tumours and in particular giant cell tumours or osteosarcomas that express the CD52 antigen. Other objects, features and advantages of the present invention will be set forth in the detailed description of preferred embodiments that follows, and in part will be apparent from the description or may be learned by practice of the invention. These objects and advantages of the invention will be realised and attained by the methods and compositions particularly pointed out in the written description and claims hereof.

This object of the present invention is achieved by the use of a CD52-specific antibody for the preparation of a medicament for the treatment of CD52 expressing solid bone tumours. Surprisingly, it could be found that certain solid bone tumours and in particular giant cell tumours or osteosarcomas express the cellular antigen CD52, which in leukaemia is already used as a target for therapy (see above). Therefore, the present invention extends this uses to cancers that are currently difficult to treat and/or can not be treated at all.

The effectiveness of a specific antibody for the antigen CD52 for solid bone tumours and in particular giant cell tumours or osteosarcomas is particular surprising, since CD52 in these cells is almost exclusively present as an intracellular antigen.

One major advantage of the use according to the present invention is based on the fact that the effect of the CD52-interacting specific antibody on the tumour cell is independent from the speed and/or amount of cellular proliferation activity. Preferably said CD52 expressing solid bone tumours are therefore selected from the group of slowly proliferating bone tumours. Particularly preferred are CD52 expressing solid bone tumours that are selected from the group of giant cell tumours, resting solid tumours (e.g. by chemotherapy), and/or osteosarcomas (although the latter do not proliferate at a slow rate).

According to a preferred aspect of the present invention, the ligand can be selected from a CD52-specific antibody, and a fragment thereof.

As already mentioned above, currently CD52 is already used as a target for therapy in leukaemia. Here, the ligand is alemtuzumab (Campath-1H, see above). The leukaemia therapy based on Alemtuzumab is well characterised, and the person of skill in the art will be readily able to amend the described therapeutical regimen in order to treat other CD52-expressing solid bone tumours, such as giant cell tumours, resting solid tumours and/or osteosarcomas. Examples of the respective literature are Moreton P and Hillmen P (Alemtuzumab therapy in B-cell lymphoproliferative disorders. Semin Oncol. 2003 Aug;30(4):493-501. Review.); Cao TM and Coutre SE (Management of advanced chronic lymphocytic leukemia. Curr Hematol Rep. 2003 Jan;2(1):65-72. Review.); Lynn A et al. (Treatment of chronic lymphocytic leukemia with alemtuzumab: a review for nurses. Oncol Nurs Forum. 2003 Jul-Aug;30(4):689-94. Review.); Stull DM (Targeted therapies for the treatment of leukemia. Semin Oncol Nurs. 2003 May;19(2):90-7. Review.); Dearden CE Alemtuzumab in lymphoproliferate disorders. Rev Clin Exp Hematol. 2002 Dec;6(4):435-48; discussion 449-50. Review.); Frampton JE and Wagstaff AJ (Alemtuzumab. Drugs. 2003;63(12):1229-43; discussion 1245-6. Review.); Cao TM and Coutre SE (T-cell prolymphocytic leukemia: update and focus on alemtuzumab (Campath-1H). Hematology. 2003 Feb;8(1):1-6. Review.), and Tallman MS (Monoclonal antibody therapies in leukemias. Semin Hematol. 2002 Oct;39(4 Suppl 3):12-9. Review.) and the respective literature cited therein. Furthermore, variants of alemtuzumab can also be used for the treatment according to the present invention, as they exhibit improved properties (Hutchins JT, et al. Improved biodistribution, tumor targeting, and reduced immunogenicity in mice with a gamma 4 variant of Campath-1H. Proc Natl Acad Sci U S A. 1995 Dec 19;92(26):11980-4.). CAMPATH-1M is a rat IgM monoclonal antibody which has been used extensively in vitro for purging bone marrow harvests in order to deplete the T cell population prior to bone marrow transplantation. Marked reduction in the incidence and severity of graft-versus-host disease has been seen with this therapy. CAMPATH-1G is a rat IgG2b class-switch variant of an IgG2a antibody recognising the CDw52 antigen which has been used in vivo to achieve immunosupression in more than 100 patients undergoing organ and bone marrow transplantation, management of organ rejection and treatment of haematologic malignancies with a high level of success. However, the rapid development of an anti-rat immunoglobulin response, including the possibility of anaphylaxis, is likely to limit the use of rat monoclonal antibodies against the CDw52 antigen in humans in vivo. CAMPATH-1H is a genetically manipulated IgG antibody obtained by grafting the complementarity determining regions from CAMPATH-1G into human framework regions. The resulting "humanised" antibody is highly effective in vitro being equivalent to the rat monoclonal antibody at complement lysis and two to four times better in cell-mediated lysis of human lymphocytes. No limiting anti-globulin response is anticipated with this humanised antibody.

In another particularly preferred embodiment of the present invention, the antibody is administered systemically and/or administered locally. The local administrating has the advantage of reduced side effects by an at the same time increased effective amount of antibody that is present at the tumour site. Subcutaneous administration of alemtuzumab has been described as a key advancement in the treatment of CLL. A phase II trial of subcutaneous alemtuzumab has demonstrated an 87% response rate in 38 previously untreated patients, with a reduction in intravenous administration-related rigors, as well as the elimination of nausea, dyspnea, diarrhoea, and hypotension, frequently seen following intravenous administration of alemtuzumab. Furthermore, encouraging results with the combination of alemtuzumab and fludarabine, which demonstrate eradication of malignant cells in patients who are resistant to either agent alone, were described to open the way for such combinations to produce durable responses even in refractory disease (Rai K, Hallek M. Future prospects for alemtuzumab (MabCampath). Med Oncol. 2002;19 Suppl:S57-63.). This subcutaneous administration of alemtuzumab can be taken as an example to readily apply such route of administration for the treatment of tumours according to the present invention.

The dosage of the anti-CD52 antibody, preferably CAMPATH-1H, to be administered to a patient suffering from solid tumours will vary with the precise nature of the condition being treated and the recipient of the treatment. The dose will generally be in the range 1 to about 100 mg for an adult patient, usually administered daily for a period between 1 and 30 days. The preferred daily dose is 1 to 10 mg per day although in some instances larger doses of up to 40 mg per day may be used. Preferably the dosage will be applied in such a manner that the antibody is present in the medicament in concentrations that provide in vivo concentrations of said antibody in a patient to be treated of between 0.01 mg/kg/day and 1 mg/kg/day.

The anti-CD52 antibody, particularly CAMPATH-1 H will generally be administered to the patient in the form of a pharmaceutical formulation. Such formulations preferably include, in addition to the antibody, a physiologically acceptable carrier or diluent, possibly in admixture with one or more other agents such as other antibodies or drugs, such as an antibiotic. Suitable carriers include, but are not limited to, physiological saline, phosphate buffered saline, phosphate buffered saline glucose and buffered saline. Alternatively the antibody, may be lyophilised (freeze dried) and reconstituted for use when needed by the addition of an aqueous buffered solution as described above. Routes of administration are routinely parenteral, including intravenous, intramuscular, subcutaneous and intraperitoneal injection or delivery. The administration can be systemic and/or locally.

The anti-CD52 antibody, may be administered in combination with or sequentially with a safe and effective amount of other drugs, in particular other drugs conventionally used in the treatment of solid tumours, or other medicaments for the alleviation of specific symptoms of the disease. Such drug is preferably selected from the group consisting of a DNA-interactive agent, alkylating agent, antimetabolite, tubulin-interactive agent, and a hormonal agent, and is, for example, selected from the group consisting of Methotrexate, Fluorouracil, Fluorode-oxyuridin, CB3717, Azacitidine, Floxuridine, Mercapyopurine, 6-Thioguanine, Pentostatin, Cytarabine, and Fludarabine. Additional chemotherapeutic agents are well-known to the skilled artisan in the field of cancer therapy. Sequential administration of the antibody with another drug may be appropriate in some cases (see, for example, Montillo M, et al. Successful CD34+ cell mobilisation by intermediate-dose Ara-C in chronic lymphocytic leukemia patients treated with sequential fludarabine and Campath-H. Leukemia. 2003 Oct 30).

Described is an improved method for screening for CD52 ligands comprising the steps of: a) incubating a cell expressing CD52 with a putative ligand, b) measuring, if a binding between CD52 and said putative ligand occurs, c) in the case of a binding of said ligand to CD52 is measured, measuring, if said binding between CD52 and said identified ligand leads to a CD52-mediated death of a CD52-expressing solid tumour cell.

In one embodiment, these ligands can be identified based on the structural similarities of CD52 with other proteins, such as B7-Ag, gp20 or CD24. Examples for ligands of these small glycosylphosphatidylinositol (GPI) anchored peptides are P-selectin and/or glycans as potential recognition signals (Aigner S, et al. CD24, a mucin-type glycoprotein, is a ligand for P-selectin on human tumor cells. Blood. 1997 May 1;89(9):3385-95.; Tone M, et al. Structure and chromosomal location of mouse and human CD52 genes. Biochim Biophys Acta. 1999 Sep 3;1446(3):334-40.).

During the course of the assay as described, the ligand will initially bind or attach to CD52. The ligand can either bind directly or indirectly to CD52, i.e. via cofactors that can be present, such as certain ions or protein factors, that promote the attachment of the ligand to CD52 and therefore support the function of the ligand. "Binding" can occur via a covalent or noncovalent attachment of the ligand or group of ligands to CD52. Based on the binding properties of the screened ligands, a first pre-selection of ligands can be performed, in which a non-binding ligand is screened in a second "round" of screening using a set of co-factors. If still no binding occurs, the ligand will be classified as "non-binding" and disregarded in further screenings. Such pre-selection will be encompassed by the terms "screening", "measuring" and/or "determining" in the general context of this invention. A ligand that shows an in-vitro modulating (e.g. lytic) action should in vivo preferably not further interact with components of the patients' or test (model) organisms' body, e.g. within the bloodstream, lung and/or heart of the patient or test organism.

In general, assays to determine a binding and biological effect of a ligand to a specific target (in this case CD52) are well known to the person skilled in the art and can be found, for example, in US patents 4,980,281, 5,266,464 and 5,688,655 to Housey (which are herein incorporated by reference) for phenotypic changes of cells after incubation with a screening ligand. Furthermore, US 5,925,333 to Krieger at al. (which is herein incorporated by reference) describes methods for modulation of lipid uptake and related screening methods.

Suitable tests for showing a biological effect of a ligand for CD52 include lytic assays that measure the release of intracellular contents (uric acid, potassium, phosphorus) into the extracellular compartment, fluorescence based assays (e.g. use of confocal fluorescent microscopy), viable cell counts, cellular proliferation assays, such as the BrdU proliferation assay or measuring of cellular calcium, and the like.

The method of screening as described can be performed in several different formats. One embodiment is a method, wherein the assay is performed in vitro. The screening assays as described preferably involve the use of bone-cancer cells and other cells, as long as these cells express CD52. How to produce such recombinant cells is well known to the skilled artisan and is further described in the respective literature.

The ligands can be initially screened using an assay such as one of the assays described herein and then tested in, for example, transgenic, CD52-expressing and/or over-expressing, animals made using standard transgenic animal technology. A technique such as embryonic stem cell technology using rats, mice or hamsters is preferred.

An additional embodiment relates to a method wherein the assay is performed in vivo. Preferably, the assay is performed in a mouse or rat. In general, the in vivo assay will not be substantially different from the above-mentioned in vitro assay. In a general screening assay for ligands of CD52 will be provided in that the ligand to be tested is/are administered to a mouse or a rat. Then, it will be determined, if said ligand leads to a CD52-mediated death of a CD52-expressing solid tumour cells compared to the absence of the ligand to be tested, wherein a difference in the death of cells identifies a ligand which leads to a CD52-mediated death of a CD52-expressing solid tumour cells of said mouse or rat. Of course, these assays can be performed in other non-human mammals as well.

An additional embodiment relates to a method according to the invention, wherein said ligand is selected from a library of naturally occurring or synthetic compounds which are randomly tested for binding to CD52. Such libraries and the methods how to build up such a library, as well as methods for using these libraries for the screening of candidate ligands are well known to the person skilled in the art and further described in the respective literature. Furthermore, some of these libraries are commercially available. The present disclosure contemplates high throughput screening of ligands for CD52. The ligands as described above, and modifications of said ligands, including analogues, derivatives, fragments, active moieties, and the like, may be screened using methods and systems as described.

In the context of the present invention, a "CD52-mediated death" encompasses all biological effects within a cell that are mediated an/or initiated by a binding of an antibody of CD52 and lead to a negative effect for the viability and/or proliferation of the cell or tissue that expresses CD52. Without limitation, such negative effects include the lysis of the cell, apoptosis and/or inhibition of the proliferative activity of the cell. Preferably, the effect is the CD52-mediated lysis of the cell (for references, see above).

Further described is a method for the production of a pharmaceutical formulation, comprising the steps of: a) performing a method as given above, and b) formulating the identified ligand for CD52 with pharmaceutically acceptable carriers and/or excipients. Such formulations therefore include, in addition to the ligand/antibody, a physiologically acceptable carrier or diluent, possibly in admixture with one or more other agents such as other antibodies or drugs, such as an antibiotic. Suitable carriers include, but are not limited to, physiological saline, phosphate buffered saline, phosphate buffered saline glucose and buffered saline. Alternatively the ligand, e.g. the antibody, may be lyophilised (freeze dried) and reconstituted for use when needed by the addition of an aqueous buffered solution as described above. Routes of administration are routinely parenteral, including intravenous, intramuscular, subcutaneous and intraperitoneal injection or delivery. The administration can be systemic and/or locally.

Finally, the present disclosure provides novel methods for the treatment of solid and particularly bone-related tumours and in particular giant cell tumours or osteosarcomas that express CD52 by the application to a patient in need thereof of an effective amount of a ligand, in particular an inhibitor, of the antigen CD52 in order to induce the CD52-mediated cellular death of the cancer cell that form the cancer to be treated. The major advantages of this novel therapy are its application in areas of the body where a surgical intervention is impossible, in the case of a multifocal disease statuses, and the application in slowly proliferating cancers (such as giant cell tumours) where current chemotherapeutical approaches can not be employed or are not effective. Furthermore, the spectrum of side effects of the treatment based o CD52 is regarded as low (Lockwood CM, Hale G, Waldman H, Jayne DR. Remission induction in Behcet's disease following lymphocyte depletion by the anti-CD52 antibody CAMPATH 1-H. Rheumatology (Oxford). 2003 Aug 29):

The present disclosure provides a method of treatment of a human subject suffering from a solid tumour that expresses the CD52 antigen, such as certain bone-related tumours, such as osteosarcomas and giant cell tumours, which comprises administering to the said subject an effective amount of an antibody recognising the CD52 antigen. Suitable formulations, routes of administrations and dosages are indicated above and are further laid out in the following examples. Further examples are described in, for example, Keating MJ, et al. (Campath-1H treatment of T-cell prolymphocytic leukemia in patients for whom at least one prior chemotherapy regimen has failed.J Clin Oncol. 2002 Jan 1;20(1):205-13.); Montillo M, et al. (Safety and efficacy of subcutaneous Campath-1H for treating residual disease in patients with chronic lymphocytic leukemia responding to fludarabine. Haematologica. 2002 Jul;87(7):695-700).

In summary, the finding of the present invention that CD52 is expressed in certain bone-related tumours, such as osteosarcomas and giant cell tumours, will form the basis for the extension of an anti-cancer therapy based on CD52-specific antibodies such as alemtuzumab (Campath-1H). The major advantages of this novel therapy are its application in areas of the body where a surgical intervention is impossible, in the case of a multifocal disease status and the application in slowly proliferating cancers (such as giant cell tumours) where the current chemotherapeutical approaches can not be employed.

PHARMACEUTICAL COMPOSITION shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan, in this case an anti-cancer therapy based on CD52-specific antibodies.

TREATMENT or THERAPY shall mean a process of the reversing or ameliorating or reducing a condition of a certain type or certain types of cells or tissues with respect to an abnormal behaviour, such as a proliferative disease. Preferably, a treatment is applied to a patient.

In the context of the present invention, the term "osteosarcoma" encompasses both osteosarcomas and chondrosarcomas, i.e. these terms can be used interchangeably throughout the present invention.

The present invention shall now be further characterised based on the following examples without being limited thereto with reference to the accompanying figures, in which
Figure 1 shows the immunohistochemical analysis of the CD52 expression a in giant cell tumours, and
Figure 2 shows the immunohistochemical analysis of the CD52 expression in an osteosarcoma.

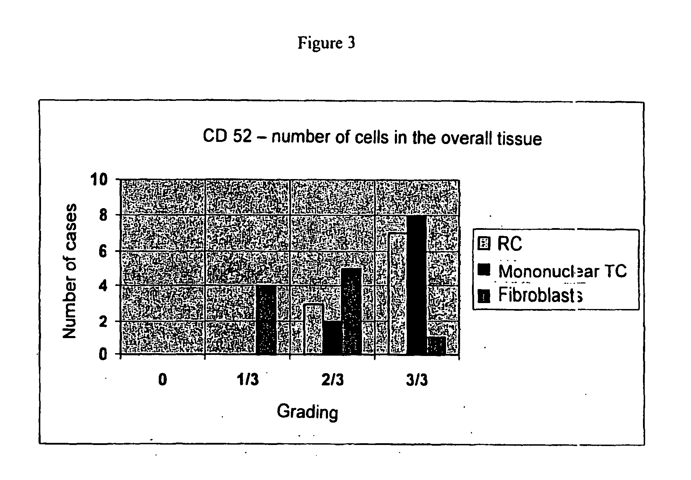

Figure 3 shows the proportion of the individual cells of the total amount of cells (TC = tumour cells; GC = giant cells of the giant cell tumour)
Figure 4 shows the proportion of the CD52-positive cells (TC = tumour cells; GC = giant cells of the giant cell tumour; further = lymphocytes and granulocytes; the proportion of these cells was below 33 % of the total amount of cells)

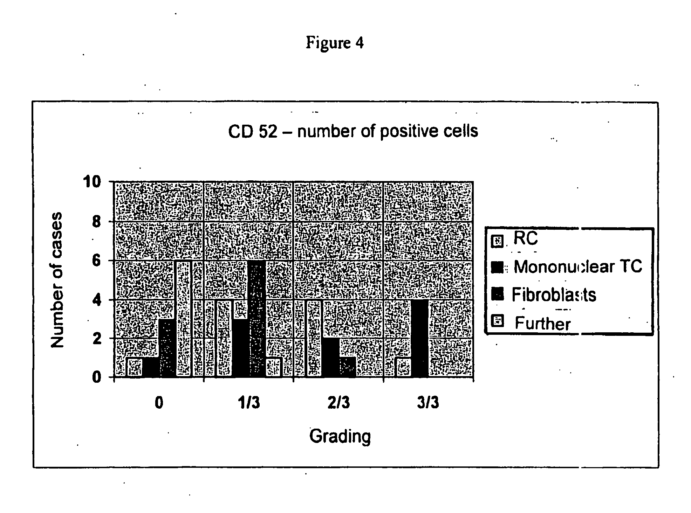

Figure 4 shows the expression of CD52 in osteosarcoma, in particular in AOB/AOC (AOB: atypic osteoblasts, AOC: atypic osteocytes)

### Examples

### Immunohistochemical analysis of the CD52 expression in giant cell tumours

For the immunohistochemical analysis of CD52 on giant cell tumours using the doubled APPAP-method of the commercially available Dako-Production kits, 1 µm thickness paraffin sections were made on poly-L-Lysin coated object slides (OS). The slides were incubated over night at 58°C. On the next day, the de-paraffination occurs by means of a serial alcohol treatment, wherein the following steps were performed: 10 minutes xylol, 5 minutes xylol, two times 5 minutes alcohol abs., 2 times 5 minutes 96 % alcohol, two times 5 minutes 70 % alcohol and subsequently 2 times 5 minutes aqua dest. After this treatment, the OS are put in PBS-2 % BSA (rinsing buffer). A proteolytic treatment with proteinase K for 5 minutes is performed. Subsequently, the incubation of the primary antibody (rat anti human, Serotec MCA 1642, 1:40) is performed for 30 minutes with subsequent rinsing for two times for 3 minutes in the buffer. The identical time periods are used for the secondary antibody (rabbit anti rat, Dako Z 0455, 1:50) and the APPAP-complex (rat, Dako D 0488, 1:100), wherein after each step, a rinsing for two times with buffer for 3 minutes is done. Due to the fact that this method is the doubled APPAP, another incubation for 10 minutes is performed using first the secondary antibody and subsequently the APPAP-complex. After rinsing for two times for 3 minutes, the chromogen is added (Fuchsin⁺, Dako K 0625) for 10 minutes which is washed away with water after the incubation period. The staining of the nucleus occurs for 5 minutes with hematoxylin. Subsequently, the nucleus is blue-dyed for 5 minutes and the OS covered with Aquatex.

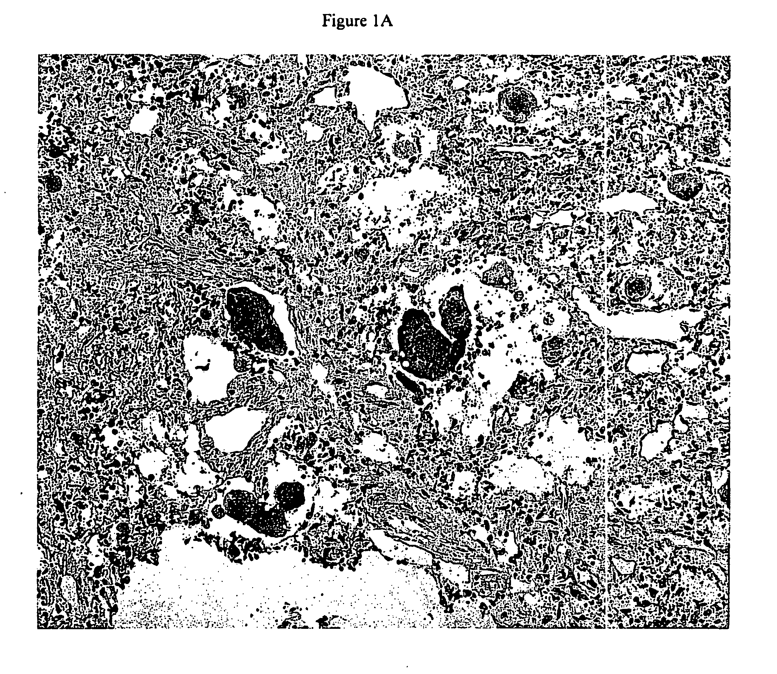

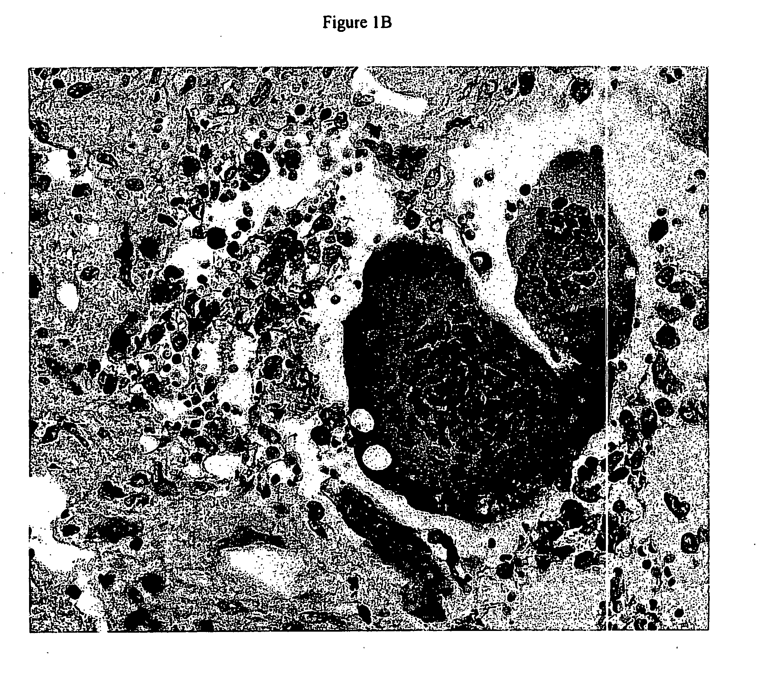

In an immunohistochemical analysis of the CD52 expression in giant cell tumours (9 cases of giant cell tumours), the expression of CD52 could be verified (see Figure 1). The CD52 expression could be identified in two cellular components of the tumour, i.e. the mononuclear cells and in the multinuclear giant cells of the tumour. The level of expression is high (compared with lymphocytes) and can be found in about 90% of the tumour cells. CD52 expression could be almost exclusively found in the cytoplasm of the tumour cells.

### Immunohistochemical analysis of the CD52 expression in osteosarcoma tumours

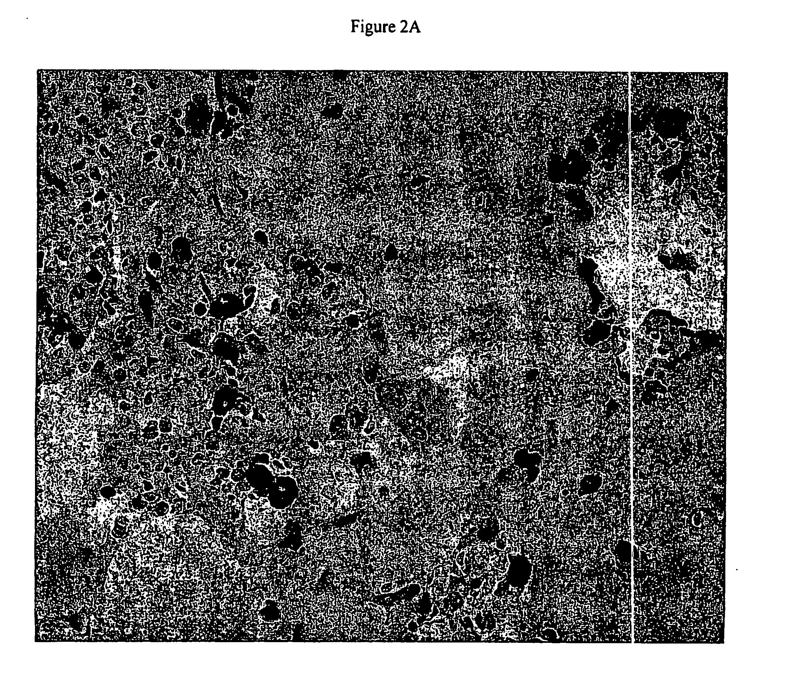

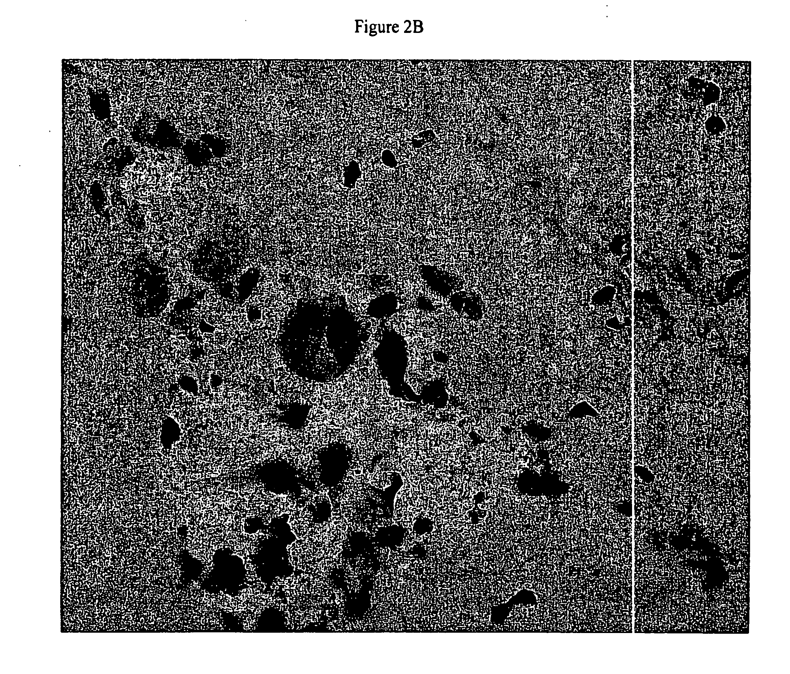

In an immunohistochemical analysis of the CD52 expression in osteosarcoma tumours (see Figure 2) and chondrosarcoma (see table 1), the expression of CD52 could be verified. The level of expression is high (compared with lymphocytes) and can be found in about 50% of the tumour cells. CD52 expression could be almost exclusively found in the cytoplasm of the tumour cells.

**Table 1: patient data OS**

| **Case** | **Gender** | **Age** | **Tissue** | **Diagnosis** |
|---|---|---|---|---|
| **RT13** | w | 15 | prox. edge of resection; dist. femur left | highly malign osteo-, chondroplastic OS (COSS RG V) |
| **RT15** | m | 74 | prox. resection ; left arm | highly malign OS (COSS RG V) |
| **RT16** | m | 40 | resection left lower lobe lung | Osteo-, chondroplastic pulmonal metastases after OS |
| **RT17** | m | 59 | prox. femur right | Fibro-, osteo-, chondroplastic OS; necrotic pulmonal tumour metastasis |
| **RT18** | w | 22 | lung metastasis segm. 9 | Pulmonal metastasis of a highly malign, osteo-, chondroplastic OS |
| **RT19** | w | 22 | lung metastasis segm. 9 | Pulmonal metastasis of a highly malign, osteo-, chondroplastic OS |
| **RT24** | m | 70 | lung metastasis right lower lobe | lung metastasis of a pre-diagnosed OS |
| **RT26** | m | 61 | amputation interthorakoscapularis left | extended highly malign Osteoplastic OS |
| **RT32** | m | 35 | dist. Femur right | multifocal differentiated paraossal OS |
| **RT33** | m | 66 | dist. Femur right | OS |

**Table 2: patient data RZT**

| **Case** | **Gender** | **Age** | **Tissue** | **Diagnosis** |
|---|---|---|---|---|
| **RT1/2** | m | 52 | right femur prox. (primary tumour/ relapse) | RZT bone |
| **RT3** | w | 23 | Distal left femur | RZT bone |
| **RT4** | m | 61 | inner pelvical resectate right, primary tumour | RZT bone |
| **RT5** | w | 27 | right radius dist. (relapse) | RZT bone |
| **RT6** | m | 69 | left pelvical resectate, primary tumour | RZT bone |
| **RT7*** | w | 64 | soft tissue retropatellar left knee (PT) | endosynovial GCT diffuse type |
| **RT8**** | w | ? | soft tissue (relapse) | RZT bone |
| **RT9*** | w | 48 | prox. Tibia left (primary tumour) | RZT bone |
| **RT10*** | m | 24 | distal femur epimethaphysis (PT) | RZT bone |
| **RT11*** | w | 47 | left hollow hand (primary tumour) | RZT bone |

### Semi-quantitative analysis

The analysis was made in a semi-quantitative fashion using a 4-graded scale, wherein 0 = no staining (0% of the cells), 1 = 1/3 staining (33% of the cells), 2 = 2/3 staining (66% of the cells), 3 = 3/3 staining(100% of the cells).

### Histopathology and immunohistochemistry GCT

For the first time, the expression of CD52 in giant cell tumour cells was detected. For this, 10 patient cases selected which were first characterised by HE-staining. The tissue consists of mononuclear tumour cells that can appear round, fusiform-like, polygonal or makrophage-like. The by far largest part is represented by osteoclast-like GC. Furthermore, necroses, macrophages and lympho-plasma cellular infiltrate can be found.

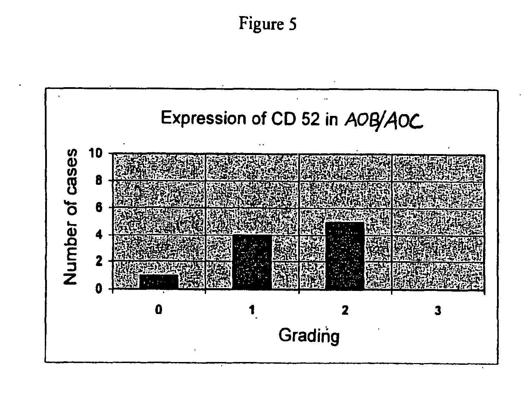

Found was the expression of CD52 in GC, wherein the expression occurs very heterogeneously (1/3 to 2/3 of the giant cells were positive in comparison to the overall number of the giant cells in the tissue; Figure 5). In 5 cases a stronger expression of CD52 could be detected, in 4 cases 1/3 was shown as CD52-positive, and in one case no expression of CD52 could be detected. Furthermore, CD52 could be detected in the mononuclear tumour cells, wherein 2/3 to 3/3 of the cells could be judged as CD52-positive (9 cases; Figure 4). 1/3 of all present fibroblasts also showed a CD52-expression. In the lymphoyctes and granulocytes, a positive reaction to the CD52-antigen could be detected only in one case.

### Histopathology and immunohistochemistry OS

The expression of CD52 in osteosarcomas (and chondrosarcomas) was detected. For this, 10 patient cases were selected, which were first characterised by HE-staining (Table 3). Subsequently, the immunohistochemical staining of the CD52-antigen (Table 3) occurred. A clearly dominant expression of CD52 could be detected in the osteoblasts (Figure 5). If lymphocytes were present, these were strongly stained in 100 %. Further CD52-positive cells were TGC and also single chondrocytes. Furthermore, also cells of the connective tissue exhibited a positive reaction towards the examined CD-antigen.

**Table 3: Histopathology and Immunohistochemistry**

| (AOS Ch: atypic chondrocytes; FB: fibroblasts; FC: fibrocytes; Ly: lymphocytes; AOB: atypic osteoblasts; AOC: atypic osteocytes; TGC: tumour giant cells;: TC: tumour cells) | | | |
|---|---|---|---|
| **Case** | **Microscopy** | **Primary cells** | **CD52-expression** |
| **RT13** | extraossous and peripheral tumour parts vital, here round to polygonal TC (basophilic or light cytoplasm; nuclei round to oval; prominent nucleoli), few osteoid; large areas with chondroid matrix (TZ chondroid differentiated, nuclei atypical) | osteoid and chondroid matrix; tumour partially limited by capsule-like CT; Ly-aggregations and single Ly. | FB/FC: 1/3 + s AOB/AOC: 1/3+ m Ch: - Ly: 3/3 + s |
| **RT15** | TC small to medium size, Nuclei oval and irregularly shaped; at the edge capsule-like CT | few osteoid matrix | AOB/AOC: - |
| **RT16** | TC polygonal with basophilic oder eosinophilic cytoplasm; nuclei of- | osteoid and chondroid matrix | AOB/AOC: 1/3 + w |
| | ten eccentric, polymorphic, hyper-chromatic with often prominent nucleoli | | Ch: 1/3+ w |
| **RT17** | vital parts: fusiform cells with polymorphism of the nuclei (long-ish); partially trabecular osteoid; chondroid parts low on cells; extended fibrosation; intraossous part: devital | fibroplastic, osteoplastic, chondroid matrix numerous Ly present | FB/FC: 2/3 + s AOB/AOC: 2/3 + s Ch: > 1/3 + w ! large Ch strongly + Ly: 3/3 + s |
| **RT18** | TC are different in size, eosinophilic cytoplasm, hyper-chromatic nuclei; some TGC having several nuclei; osteoid matrix with centralised devital tumour tissue | osteoid matrix numerous Ly present | TGC: 2/3 + s AOB/AOC: 2/3 + s Ly: 3/3 + s |
| **RT19** | TZ fusiform to polygonal (eosinophilic or basophilic cytoplasm), hyperchromatism and prominent nucleoli; TRC present; osteoid and chrondroid matrix; small areas of devitalised parts; at the edge BG | osteoid, chondroid matrix with fibroplastic parts | AOB/AOC: 2/3 + w Ch: 2/3 + w TGC: - FB/FC:- |
| **RT24** | fusiform TC, loose chromatin with prominent nucleoli; fibroplastic parts | osteoid matrix with fibroplastic, chondroplastic and inflammatory parts | AOB/AOC: 2/3 + m Ly: 3/3 + s FB/FC: 1/3 + s Ch: very large Ch + m, residual |
| **RT26** | polygonal and single fusiform TC (eosinophilic cytoplasm); nuclei partially eccentric, round to oval; prominent nucleoli; osteoid | mainly osteoid matrix, single Ly | AOB/AOC: 2/3 + m Ly: 3/3 + m |
| **RT32** | In the osteoid few polymorphic TC; intertrabecular fusiform TC, in part fiborblast-like; herd-like necroses; few chondroid matrix | osteoid matrix with chondroid parts | fibroblast-like TC to 1/3 w + identical for AOB/AOC |
| **RT33** | osteoclastic GC; larger necroses; chondroblastic part forms eosinophilic chondroid; compactly stored TC in the osteoid | chondroid and osteoid matrix | AOB/AOC: 2/3 + m TGC: 2/3 + m |

### Treatment of a solid tumour based on a CD52 specific antibody

### Treatment scheme A):

The patients (optionally including chemotherapy-naive patients) will receive 3, 10, and 30 mg of Campath-1H on sequential days, followed by 30 mg three times weekly, as 2-hour intravenous infusions, for 4 to 12 weeks. The objective response rate will be expected at about 50% (40% to 63%), with an about 40% complete response (CR) rate (28% to 51%). The most common Campath-1H-related adverse events will be expected as acute reactions during or immediately after infusions. Infectious episodes during treatment may lead to treatment discontinuation.

### Treatment scheme B):

As intravenous Campath-1H is almost invariably associated with reactions, sometimes of WHO grade 3-4, the subcutaneous route of administration will be adopted, which proves to induce rare and mild adverse reactions but usually has comparable efficacy.

The patients who, optionally, responded to FAMP will received subcutaneous Campath-1H, three times a week for 6 weeks in escalating doses up to 10 mg. The patients will receive acyclovir and cotrimoxazole as infection prophylaxis. Granulocyte colony-stimulating factor (G-CSF), at the dosage of 5-10 microg/kg/day, or intermediate-dose Ara-C (800 mg/m(2)/q 12h x 6 doses), can be administered to obtain peripheral blood stem cell (PBSC) mobilisation.

### Treatment scheme C):

The patient will received treatment with CAMPATH-1H (2 mg/day iv for five days, two days rest, then 10 mg/day iv for five days). The "double-peaked" pattern will be seen with neutrophils which increases during the same time course and coincides with the second and third dosing days at 2 and 10 mg respectively (10 mg doses will follow a two day rest).

The 10 dose CAMPATH-1H treatment course should be well tolerated. Fever and headache might be reported as adverse events with both occurring with administration of the first 2 mg and 10 mg doses.

The person of skill will be readily able to amend the above given treatment schemes with respect to other ligands, such as protein, in particular protein or peptide mimetica of anti-CD52 antibodies, glycans and P-selectin.

## Claims

1. Use of a CD52-specific antibody for the preparation of a medicament for the treatment of CD52 expressing solid bone tumours.

2. Use according to claim 1, wherein the CD52 expressing solid bone tumours are selected from the group of giant cell tumours, chondrosarcomas, and osteosarcomas.

3. Use according to claim 1 or 2, wherein the antibody is alemtuzumab (Campath-1H).

4. Use according to any of claims 1 to 3, wherein the antibody is administered systemically and/or administered locally.

5. Use according to any of claims 1 to 4, wherein the antibody is present in the medicament in concentrations that provide in vivo concentrations of said antibody in a patient to be treated of between 0.01 mg/kg/day and 1 mg/kg/day.

6. Use according to any of claims 1 to 5, wherein the antibody is administered in combination with other chemotherapeutically active substances.

## Patentansprüche

1. Verwendung eines CD52-spezifischen Antikörpers zur Herstellung eines Medikaments zur Behandlung von CD52 exprimierenden soliden Knochentumoren.

2. Verwendung nach Anspruch 1, wobei die CD52 exprimierenden soliden Knochentumore ausgewählt sind aus der Gruppe von Riesenzelltumoren, Chondrosarkomen und Osteosarkomen.

3. Verwendung nach Anspruch 1 oder 2, wobei der Antikörper Alemtuzumab (Campath-1H) ist.

4. Verwendung nach einem der Ansprüche 1 bis 3, wobei der Antikörper systemisch und/oder lokal verabreicht wird.

5. Verwendung nach einem der Ansprüche 1 bis 4, wobei der Antikörper in dem Medikament in Konzentrationen vorhanden ist die in vivo Konzentrationen des Antikörpers in einem zu behandelnden Patient von zwischen 0,01 mg/kg/Tag und 1 mg/kg/Tag zur Verfügung stellen.

6. Verwendung nach einem der Ansprüche 1 bis 5, wobei der Antikörper in Kombination mit anderen chemotherapeutisch aktiven Substanzen verabreicht wird.

## Revendications

1. Utilisation d'un anticorps spécifique de CD52 pour la préparation d'un médicament destiné au traitement de tumeurs osseuses solides exprimant CD52.

2. Utilisation selon la revendication 1, dans laquelle les tumeurs osseuses solides exprimant CD52 sont choisies dans le groupe constitué par les tumeurs à cellules géantes, les chondrosarcomes, et les ostéosarcomes.

3. Utilisation selon la revendication 1 ou 2, dans laquelle l'anticorps est l'alemtuzumab (Campath-1H).

4. Utilisation selon l'une quelconque des revendications 1 à 3, dans laquelle l'anticorps est administré de manière systémique et/ou locale.

5. Utilisation selon l'une quelconque des revendications 1 à 4, dans laquelle l'anticorps est présent dans le médicament en des concentrations qui fournissent des concentrations in vivo dudit anticorps chez un patient à traiter comprises entre 0,01 mg/kg/jour et 1 mg/kg/jour.

6. Utilisation selon l'une quelconque des revendications 1 à 5, dans laquelle l'anticorps est administré en combinaison avec d'autres substances chimiothérapeutiquement actives.
